# EUROPEAN PATENT SPECIFICATION

(11) **EP 3 698 740 B1**
(45) Date of publication and mention of the grant of the patent: **30.11.2022**
(21) Application number: 18897791.2
(22) Date of filing: 08.11.2018
(51) Int. Cl.: A61B 17/3207, A61B 17/22

(54) **THROMBUS RETRIEVAL CATHETER**
THROMBUSENTNAHMEKATHETER
CATHÉTER D'EXTRACTION DE THROMBUS

(30) Priority: 26.12.2017 CN 201711434562
(43) Date of publication of application: 26.08.2020
(73) Proprietor: Shanghai Bluevascular Medtech Co., Ltd., Shanghai 201318 (CN)
(72) Inventor: HAN, Jianchao, Shanghai 201318 (CN); ZHANG, Linlin, Shanghai 201318 (CN); FAN, Yaming, Shanghai 201318 (CN); LIU, Mengqin, Shanghai 201318 (CN); SHI, Zengzuo, Shanghai 201318 (CN); ZHU, Qing, Shanghai 201318 (CN); MIAO, Zhenghua, Shanghai 201318 (CN)
(74) Representative: Loo, Chi Ching
(86) International application number: PCT/CN2018/114551
(87) International publication number: WO 2019/128485

(56) References cited:
- WO-A1-89/00835
- CN-A- 104 758 029
- CN-A- 104 768 482
- CN-A- 108 013 918
- CN-U- 203 591 299
- CN-U- 204 158 450
- CN-U- 205 126 342
- DE-A1- 19 955 592
- US-A- 5 350 390
- US-A1- 2004 236 312
- US-A1- 2007 250 096
- US-A1- 2017 224 375

## Description

### Field of the Invention

The present invention relates to a medical catheter, in particular relates to a thrombus retrieval catheter.

### Background of the Invention and Discussion of the Prior Art

Deep vein thrombosis (DVT) is a disease caused by abnormal clotting of blood in the deep veins of the lower extremities. DVT increased venous pressure and blocked blood return, which can cause lower extremities swelling, pain, dysfunction and the risk of thrombosis. The shed thrombus can cause pulmonary embolism (PE). If DVT is not effectively treated in the acute phase, it can cause thrombosis, venous obstruction, loss of valve function, venous reflux and venous hypertension, post thrombosis syndrome (PTS) is formed, endangering limb survival and threatening life safety.

Aspirin, heparin, or warfarin etc. can effectively dissolve venous thrombosis, and greatly reduce the incidence of DVT and PE. However, there are significant limitations in the treatment, such as easy bleeding constitution, hemorrhagic diabetes, hemorrhagic stroke, neurological surgery, severe trauma, pleural hemorrhage, intracranial hemorrhage fractures of the pelvis and lower limbs, anticoagulant disorders, such conditions may cause severe body bleeding and endanger the patient's life.

Catheter-directed thrombolysis (CDT), directed delivery of thrombolytic drugs (such as urokinase, etc.) to the thrombus site, can effectively reduce the thrombus to restore the intravenous positive blood flow and remove or relieve vein obstruction, protect the venous valves shape and function, interrupt the pathological process of PTS and reduce the incidence of PTS. However, CDT treatment of DVT takes a long time (average 53.4 hours), which will cause the following problems: indwelling the catheter for a long time which increase the patient's discomfort and prolonging the hospital stay; repeated haemospasia, needing for close monitoring and high-level care. It is not conducive to opening blood flow as soon as possible for the cases with severe swelling or even threatening limb survival; bleeding risk of thrombolytic drugs; CDT is not suitable for patients with high risk of bleeding (such as severe high blood pressure, etc.), and for patients with childbirth and pregnancy.

Traditional surgical thrombectomy is suitable for DVT patients who have severe clinical symptoms and cannot use thrombolytic drugs. The disadvantages include that, as venous incision and thrombectomy are invasive procedures, they are not suitable for patients with poor health. Thrombectomy may destroy valve function. If there are residual thrombi, anticoagulation may prone to wound complications.

With the development of technology, a device for percutaneous mechanical thrombectomy (PMT) has appeared in recent years. It is a group of instruments used to thrombectomy of acute or subacute thrombosis in blood vessels. It dissolves, crushes, and sucks to remove thrombus, and restore blood circulation and valve function. PMT is a microtraumatic intraluminal thrombus removal device that can quickly remove thrombus, restore blood flow and save valve function.

WO2011/024124A1 discloses a mechanical thrombectomy device which consists of a catheter with a side hole at top, a spiral structure inside the catheter and a guide wire. The spiral structure can rotate at high speed which can form vacuum combined action of the catheter and the guide wire, the thrombus located near the side hole of the catheter is suctioned into the catheter during its operation. And the spiral structure cooperates with the side hole of the catheter to cut the inhaled thrombus to achieve the purpose of breaking the thrombus, and finally the thrombus is discharged out of the body. The speed of the spiral structure during suction is very fast (40,000 ~ 60000rpm), which will drive the guide wire to rotate at a high speed. Some blood substances (such as fibrin, floating thrombus, etc.) will be winded in the guide exposed to the blood under the action of centrifugal force. There is a risk of thrombus entangling, which can cause the guide wire to rupture. WO89/00835A1 discloses another known retrieval catheter in the field; a catheter for opening obstructions in human blood vessels and the like comprising a flexible tube equipped with a hollow annular cutter rotatably mounted at its first end and driven by a motor through an off-center flexible wire and a suction pump at its other end for extracting the obstructions severed by the annular cutter.

Therefore, it is necessary to provide a new type of thrombus retrieval catheter, which can effectively prevent the guide wire from rotating, thereby eliminating the risk of thrombus entangling and guide wire rupture.

### Summary of the Invention

The technical problem to be solved by the present invention is to provide a thrombus retrieval catheter, so that the mechanical thrombus retrieval system can fast and continuous thrombus retrieval without the problems of thrombus entangling, guide wire rupture and the like. thereby the catheter can achieve fast and continuous thrombectomy. The invention is defined by appended claim 1 and preferred embodiments are defined in the dependent claims.

The technical solution adopted by the present invention for solving the above-mentioned technical problems is to provide a thrombus retrieval catheter, according to claim 1, comprising a thrombectomy head a thrombus cutter, a transmission cable, a blocking tube, an outer sheath, and an end connecting part. The thrombectomy head has a hollow tubular structure and at least one thrombectomy hole. The thrombus cutter has a further hollow tubular structure which has at least one blade. The thrombus cutter is installed inside the thrombectomy head. The thrombus cutter is fixedly connected to a distal end of the transmission cable. A proximal end of the thrombectomy head is fixedly connected to a distal end of the outer sheath. A proximal end of the outer sheath is fixedly connected to the end connecting part. The blocking tube is provided as a sleeve on the outside of the transmission cable. A proximal end of the blocking tube is fixedly connected to the end connecting part. The thrombus cutter is provided as a sleeve on the outside of the blocking tube. The end connecting part is provided with a side hole. The side hole is in communication with a lumen formed between the blocking tube and the outer sheath.

Preferably, at least one side wing is fixedly provided on the distal end of the blocking tube, the thrombus cutter is provided as a sleeve on the outside of side wing.

Preferably, the thrombectomy hole on the thrombectomy head is completely covered by the thrombus cutter in the axial direction.

Preferably, the axial length of the side wing is not less than the axial length of the thrombus cutter.

Preferably, the number of the side wings is equal to the number of thrombectomy hole.

Preferably, the side wing provided on the blocking tube is a long and narrow sheet.

Preferably, the side wing is a spiral side wing, and a cutting edge is provided on the spiral side wing.

Preferably, a negative pressure suction device is connected with the side hole, and a motor is connected with the proximal end of the transmission cable.

Preferably, a hydrophilic coating is covered over the outer surface of the blocking tube.

Preferably, the angle of the thrombectomy hole is 60~120° for the retrieval catheter used in artery, and the angle of the thrombectomy hole is 30-60° for the retrieval catheter used in vein.

Preferably, a guide wire lumen through which a guide wire can pass is correspondingly provided between the thrombectomy head and outer sheath.

Compared with the prior art, the beneficial effects of the present invention are as follows: the thrombus retrieval catheter provided by the present invention can make the mechanical thrombus retrieval system fast and continuous thrombus retrieval without the problems of thrombus winding and guide wire rupture. Specifically, there is no guide wire in the thrombus aspiration lumen, which eliminates the risk of thrombus winding and fracturing guide wire. A blocking tube is provided on the outside of the transmission cable, which prevents thrombus from winding on the transmission cable. In particular, a side wing is provided between the thrombus cutter and the blocking tube, which effectively prevents thrombus driven by the high-speed rotating thrombus cutter from winding on the blocking tube, so as to achieve fast and continuous thrombus retrieval.

### Brief Description of the Drawings

Fig.1 is a schematic view illustrating the overall structure of the thrombus retrieval catheter according to one embodiment of this invention.
Fig.2 is a schematic view illustrating structure of the thrombus engaging head according to one embodiment of this invention.
Fig.3 is a schematic view illustrating connecting structure of the thrombus cutter and the transmission cable according to one embodiment of this invention.
Fig.4 is a schematic view illustrating structure of the blocking tube with a side wing according to one embodiment of this invention.
Fig.5 is a cross-sectional view of the end connecting part according to one embodiment of this invention.
Fig.6a-6c is a schematic view illustrating structure of the thrombectomy holes with different size according to one embodiment of this invention.
Fig.7a-7h is a schematic view illustrating structure of the thrombectomy holes with different shape according to one embodiment of this invention.
Fig.8 is a schematic view illustrating the double-lumen structure of a thrombectomy head according to one embodiment of this invention.
Fig.9 is a schematic view illustrating the double-lumen structure of the outer sheath according to one embodiment of this invention.
Fig.10 is a schematic view illustrating the blocking tube structure of two side wings according to one embodiment of this invention.
Fig. 11 is a schematic view illustrating the blocking tube structure of three side wings according to one embodiment of this invention.

In the Figures:
1000-a catheter
1100-a thrombectomy head
1110, 1111, 1112-a thrombectomy hole
1110a, 1110b, 1110c, 1110d, 1110e, 1110f, 1110g, 1110h-thrombectomy hole
1200-a-thrombus cutter
1210, 1211, 1212-a blade
1300-an outer sheath
1400-an end connecting part
1410-a side hole
1500-a transmission cable
1600-a side wing
1610-a spiral side wing
1611-a cutting edge
1620-a spiral side wing
1621-a cutting edge
1700, 1710, 1720-a blocking tube
1800-a double-lumen thrombectomy head
1810-a thrombectomy hole
1820-a guide wire lumen
1900-a double-lumen outer sheath
1910-a thrombus aspiration lumen
1920-a guide wire lumen of an outer sheath

### Detailed Description of the Invention

The invention will now be further described below with reference to the accompanying drawings and examples. In the present invention, the distal end refers to the end away from the operator (such as a doctor), the proximal end refers to the end near the operator (such as a doctor).

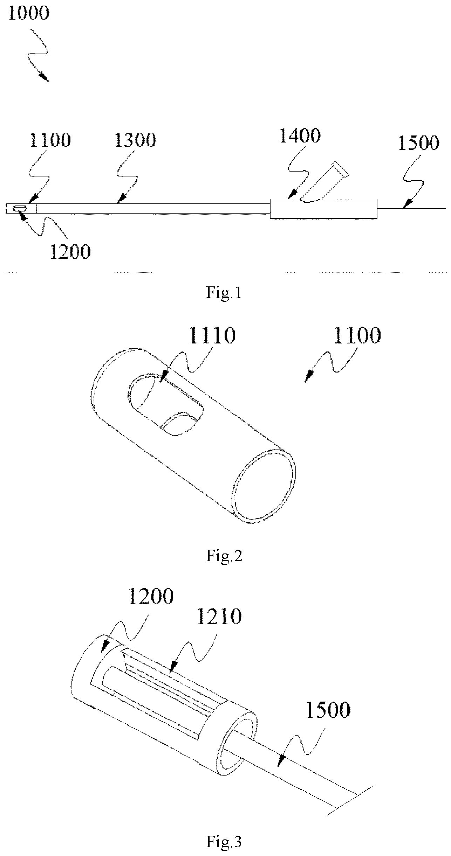

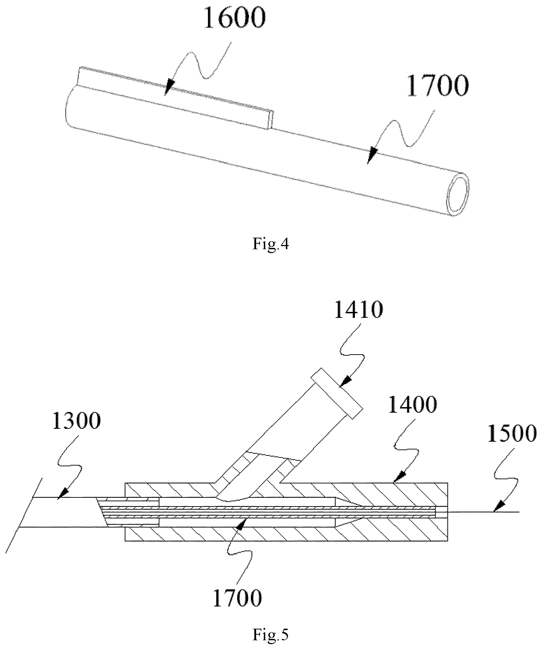

Fig.1 is a schematic view illustrating the overall structure of the thrombus retrieval catheter according to one embodiment of this invention. Fig.2 is a schematic view illustrating structure of the thrombectomy head according to one embodiment of this invention. Fig.3 is a schematic view illustrating the connecting structure of a thrombus cutter and a transmission cable according to one embodiment of this invention. Fig.4 is a schematic view illustrating structure of the blocking tube with a side wing according to one embodiment of this invention. Fig.5 is a sectional view of an end connecting part according to one embodiment of this invention.

As shown in Fig.1, a thrombus retrieval catheter provided in the embodiment, from the distal end to the proximal end, comprising a thrombectomy head 1100, a thrombus cutter 1200, an outer sheath 1300, an end connecting part 1400 and a transmission cable 1500. As shown in Fig.4, a thrombus retrieval catheter further comprising the outer sheath 1300, a side wing 1600 provided in the thrombectomy head 1100 and a blocking tube 1700. The proximal end of the thrombectomy head 1100 is connected with the distal end of the outer sheath 1300, the proximal end of the outer sheath 1300 is connected with the distal end of the end connecting part 1400. As shown in Fig.5, the proximal end of the outer sheath 1300 can be partially inserted into the lumen of the end connecting part 1400. When the transmission cable 1500 rotates, the outer sheath 1300 and the thrombectomy head 1100 do not rotate accordingly. The outer sheath 1300 and the thrombectomy head 1100 may also be integrated, that is, they are the same component.

As shown in Fig.2, the thrombectomy head 1100 has a hollow component with at least one thrombectomy hole 1110. Considering that the size of a single thrombectomy hole is sufficient to inhale the thrombus smoothly without affecting the firmness of the thrombectomy head the thrombectomy holes 1110 preferably has 2-3 thrombectomy holes 1110. The length of the thrombectomy holes 1110 is not particularly limited, but it is preferably 2 to 3mm. Because if the thrombectomy holes 1110 is too long, it means that the thrombectomy head 1100 is too large, which makes the ability of the distal end of the thrombus retrieval catheter to pass through the curved blood vessels get worse. If the thrombectomy holes 1110 is too short, it means that it is difficult for a large-sized thrombus to enter the thrombectomy head, which cannot break or suck thrombus perfectly.

As shown in Fig.3, the thrombus cutter 1200 is a hollow component with at least one blade 1210, the number of blades 1210 is preferably the same as the number of thrombectomy holes 1110, that will bring higher efficiency of breaking thrombus. The thrombus cutter 1200 is fixed at the distal end of the transmission cable 1500 and installed inside the thrombectomy head 1100, and completely covers the thrombectomy holes 1110 in the axial direction (that is, the axial length of the thrombus cutter 1200 is not less than the axial length of the thrombectomy holes 1110), so that at least one blade 1210 of the thrombus cutter 1200 can be exposed from the thrombectomy holes 1110 when the blade 1210 of the thrombus cutter 1200 rotates to a certain position. The transmission cable 1500 is made of single strand of wire or multiple strands of wires, which is connected with a motor (not shown in the Figure). The thrombus cutter 1200 is driven to rotate when the motor is started. At the same time, when the transmission cable 1500 rotates, the outer sheath 1300 and the thrombectomy head 1100 do not rotate with it, that results in the relative rotation of the thrombus cutter 1200 and the thrombectomy head 1100, and case relative movement of the blade 1210 and the edge of the thrombectomy holes 1110. So that, the blade 1210 can cut the object approaching or entering the thrombectomy head 1110, so as to achieve the purpose of breaking thrombus.

As shown in Fig.4, the blocking tube 1700 is a thin-walled hollow tubular or a spiral hollow tubular made of multiple strands of wires, such as a hypo tube. the proximal end of the blocking tube 1700 is connected to the end connecting part 1400. As shown in Fig. 5, the proximal end of the blocking tube 1700 can be inserted into the lumen of the end connecting part 1400, the proximal end of the blocking tube 1700 is clamped by the inner wall of the end connecting part 1400, so as to keep it fixed. The blocking tube 1700 is provided as a sleeve on the outside of the transmission cable 1500, at the same time the thrombus cutter 1200 is provided as a sleeve on the outside of the blocking tube 1700. When the thrombus cutter 1200 is driven by the transmission cable1500 to rotate, since the blocking tube 1700 is not connected to the transmission cable 1500 or the thrombus cutter 1200, the blocking tube 1700 does not rotate accordingly. The side wing 1600 is a long and narrow sheet which is fixedly provided at the distal end of the blocking tube 1700 and in the thrombus cutter 1200. In one embodiment, the side wing 1600 is a long and narrow plane which is the plane of the rotation axis of the transmission cable 1500 located. In other words, from the cross section of the blocking tube 1700, the extension line of the side wing passes through the axis of the blocking tube 1700. The blocking tube 1700 has at least one side wing 1600, preferably 2 to 3 side wings 1600, and more preferably the number of side wings 1600 is the same as the number of thrombectomy holes 1110, which can make the side wings 1600 more effectively block the winding of thick objects such as thrombus. The inner diameter of the side wings 1600 and the thrombus cutter 1200 are precisely matched, so that the diameter gap between the side wings 1600 and the thrombus cutter 1200 is kept as small as possible. Due to the existence of the side wings 1600, the annular cavity between the inner wall of the thrombus cutter 1200 and the outer wall of the blocking tube 1700 does not connected through in the circumferential direction. For example, if there are two side wings 1600, the annular lumen in the thrombus cutter 1200 is divided into two lumens in the circumferential direction; if there is only one side wing 1600, the annular cavity in the thrombus cutter 1200 is divided into one lumen with starting point and ending point in the circumferential direction. For better separation, the axial length of the side wing 1600 is not less than the axial length of the thrombus cutter 1200, 0 to 1mm longer than the thrombus cutter 1200 is preferable. This is because the rotation of the thrombus cutter 1200 will cause the blood containing thrombus in the thrombus retrieval catheter to rotate in a vortex shape. During the movement of the blood toward the proximal end of the thrombus retrieval catheter, the blood will continue to rotate for a certain distance. Therefore, if the side wing 1600 that plays a role in separation is as long as or slightly longer than the thrombus cutter 1200, it can better block the winding of blood viscous material. Preferably, the shape of the side wing 1600 is an elongated rectangle, or other shapes close to the elongated rectangle.

The thrombectomy head 1100 and the thrombus cutter 1200 are made of sintered ceramic, cermet, titanium alloy, stainless steel, tungsten steel or other metals. The transmission cable 1500, the side wing 1600, and the blocking tube 1700 are made of metal such as titanium alloy, stainless steel, or tungsten steel. The outer sheath 1300 is a single-layer or multi-layer composite tube, the distal end of the outer sheath 1300 is connected to the proximal end of the thrombectomy head 1100, the distal end of the out sheath 1300 is connected to the distal end of the end connecting part 1400. A side hole 1410 is provided in the end connecting part 1400, the side hole 1410 is connected to the lumen formed by the blocking tube 1700 and the outer sheath 1300.

The thrombectomy head 1100, the thrombus cutter 1200, the transmission cable 1500, the side wing 1600, the outer sheath 1300 and the end connecting part 1400 are made by mechanical processing, injection (extrusion) molding, laser engraving and other processing methods. As shown in Fig.3, the distal end of the transmission cable 1500 is fixed inside the closed end of the thrombus cutter 1200 by laser welding. As shown in Fig.4, the side wing 1600 is fixed on the blocking tube 1700 by laser welding. As shown in Fig.2, the fixed thrombus cutter 1200 is put into the thrombectomy head 1100. As shown in Fig.3, the blocking tube 1700 fixed with the side wing 1600 is put into the hollow tubular lumen of the thrombus cutter 1200. As shown in Fig.5, the distal end of the outer sheath 1300 is connected to the proximal end of the thrombectomy head 1100 by using glue or other means of connection. The proximal end of the outer sheath 1300 is connected to the end connecting part 1400, the proximal of the blocking tube 1700 is fixed in the end connecting part 1400.

Before the operation, immerse the thrombectomy head 1100 of the thrombus retrieval catheter 1000 in saline. As shown in Fig.5, using the syringe to suck the side hole 1410 of the end connecting part 1400 until the saline can be seen in the syringe, then stop sucking and complete the system evacuation. During the operation, puncture the blood vessel and deliver the thrombus retrieval catheter 1000 to the lesion (thrombus) site, using a 50ml syringe (additional configuration) connect the side hole 1410 for suctioning and driving the thrombus cutter 1200 to rotate by the motor (additional configuration) through the transmission cable 1500, the blocking tube 1700 and the thrombectomy head 1100 do not rotate. So the rotating blade 1210 and the relatively stationary thrombectomy hole 1110 can cut and smash the thrombus sucked into the thrombus retrieval catheter 1000, under the protection of the blocking tube 1700, the crushed blood substances such as thrombus and fibrin will not adhere to the rotating transmission cable 1500, which prevents the rotation of the transmission cable 1500 from being hindered. At the same time, under the obstruction of the side wing 1600, blood substances such as crushed thrombus and fibrin are not easily entangled on the blocking tube 1700. This is because the annular lumen in the thrombus cutter 1200 is separated by the side wings 1600, and is no longer an annular lumen connected through in the circumferential direction, so that the viscous material is difficult to be entangled. At the same time due to the vacuum suction at the side hole 1410, the viscous material is sucked away before being entangled and attached, and they will not stay in the distal lumen. After working for a period of time, the thrombus mixture in the 50ml syringe can be poured out for multiple suctions according to the pathological condition. After the thrombus is suctioned, turn off the motor and withdrawn all devices from the human body.

Alternatively, a peristaltic pump with a suction capacity of 30-200ml/min can be connected to the side hole 1410 for thrombus suction.

Alternatively, a metal or non-metal coating (such as a hydrophilic coating) is added to the outer surface of the blocking tube 1700 to prevent the suctioned thrombus from adhering to the outer surface of the blocking tube 1700.

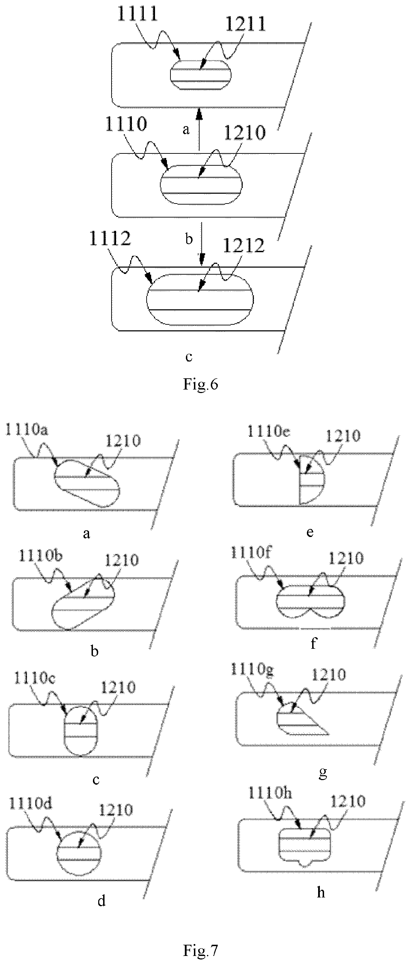

Alternatively, as shown in Fig.6, the size of the thrombectomy hole 1110 can be adjusted, the corresponding length adjustment of the side wing 1600 and the thrombus cutter 1200 to meet different needs of thrombus taking. Preferably, a larger thrombectomy hole 1112 is used in the artery to improve the efficiency of thrombus extraction, and a smaller thrombectomy hole 1111 is used in the vein to protect the vein wall. For thrombectomy catheter apply to artery, the angle of the thrombectomy hole is preferably 60-120°, and for thrombus retrieval catheter suitable for vein, the angle of the thrombectomy hole is preferably 30 ~ 60 °. The angle of the thrombectomy hole refers to a central angle of the arc of the thrombectomy hole on the cross section of the thrombectomy head 1100 (the section perpendicular to the axial direction).

In other embodiments, as shown in Fig.7, the thrombectomy hole 1110 can also be different configurations, as shown in Fig.7a, 7c and 7e, the middle of the thrombectomy hole 1110a,1110b,1110c is rectangle and both ends of the is circular arc, its opening direction can be set as required. The thrombectomy hole 1110d shown in Fig.7b is circular. The thrombectomy hole 1110e shown in Fig.7e is semi-circular. The thrombectomy hole shown in Fig.7f, 7g and 7h are in different shape, in this embodiment, the shape of the thrombectomy hole 1110 is not limited.

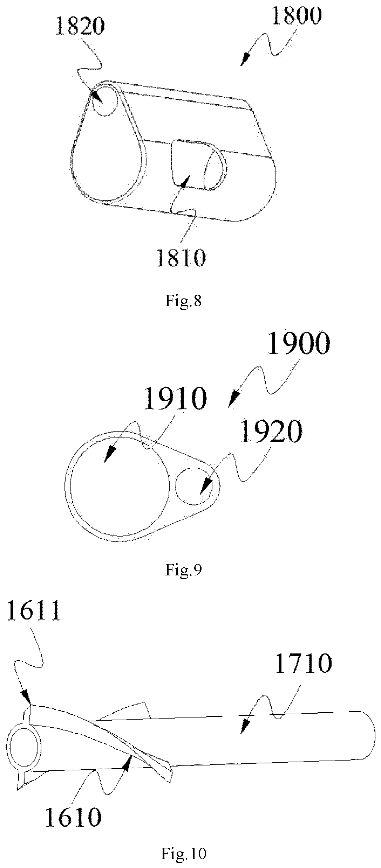

In other embodiments, as shown in Fig.8 and Fig.9, the thrombectomy head 1800 is a double-lumen structure comprising a lumen connected with the thrombectomy hole 1810 and a guide wire lumen 1820. Correspondingly, the outer sheath tube1900 is also a double-lumen structure comprising a thrombus aspiration lumen 1910 connected with the lumen of the thrombectomy hole 1810 and a guide wire lumen of the outer sheath 1920 connected with the guide wire lumen 1820. The guide wire lumen 1820 and the guide wire lumen of the outer sheath 1920 are used for the guide wire to pass through in order to better guide the thrombectomy head 1800 to the lesion location during the operation. When such a structure is adopted, the lumen where the guide wire is located and the lumen where the rotating structure is located (the transmission cable 1500, the thrombus cutter 1200, etc.) are not connected and they will not affect each other, which can effectively prevent the guide wire from rotating with the system, and then it eliminates the risk of thrombus winding the guide wire and the guide wire breaking.

In other embodiments, as shown in Fig.10, two clockwise spiral side wings 1610 are fixed on the blocking tube 1710, and the spiral side wings 1610 have a cutting edge 1611, that is, the outer side of the spiral side wings 1610 have a sharp cutting edge which can cut off the wound blood substances (fibrin, floating thrombus) for reducing the risk of thrombus winding.

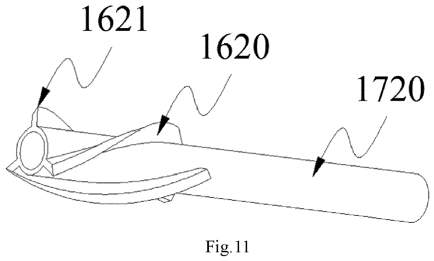

In other embodiments, as shown in Fig.11, three counterclockwise spiral side wings 1620 are fixed on the blocking tube 1720, and the outer side of the spiral side wings 1620 have a cutting edge 1621 which further reduces the risk of thrombus winding.

In summary, the thrombus retrieval catheter provided by the present invention can make the mechanical thrombectomy system fast and continuous thrombectomy without the problems of thrombus winding, guide wire rupture and the like. Specifically, there is no guide wire in the thrombus aspiration lumen, eliminating the risk of thrombus winding guide wire and guide wire breakage. A blocking tube is provided on the outside of the transmission cable, preventing thrombus from winding on the transmission cable. In particular that a side wing is provided between the thrombus cutter and the blocking tube which can effectively prevent the thrombus driven by the high-speed rotating thrombus cutter from winding on the blocking tube, thereby the catheter can achieve fast and continuous thrombectomy,

While the present invention has been disclosed as above in preferred embodiments, it is not intended to limit the invention. Any person skilled in the art may make various improvement and modifications within the scope of the invention. Therefore, the scope of protection of the invention shall be subject to that defined in the claims.

## Claims

1. A thrombus retrieval catheter (1000), comprising a thrombectomy head (1100), a thrombus cutter (1200), a transmission cable (1500), an outer sheath (1300), and an end connecting part (1400);
the thrombectomy head has a hollow tubular structure and at least one thrombectomy hole (1100, 1111, 1112); the thrombus cutter has a further hollow tubular structure which has at least one blade (1210); the thrombus cutter is installed inside the thrombectomy head; the thrombus cutter is fixedly connected to a distal end of the transmission cable; a proximal end of the thrombectomy head is fixedly connected to a distal end of the outer sheath; a proximal end of the outer sheath is fixedly connected to the end connecting part; the end connecting part is provided a side hole (1410);
**characterised in that**, the catheter further comprises a blocking tube (1700, 1710, 1720), the blocking tube is provided as a sleeve on the outside of the transmission cable; a proximal end of the blocking tube is fixedly connected to the end connecting part; the thrombus cutter is provided as a sleeve on the outside of the blocking tube; and wherein, the side hole is in communication with a lumen formed between the blocking tube and the outer sheath.

2. The thrombus retrieval catheter of claim 1, wherein at least one side wing (1600) is fixedly provided on the distal end of the blocking tube, the thrombus cutter is provided as a sleeve on the outside of side wing.

3. The thrombus retrieval catheter of claim 1, wherein the thrombectomy hole on the thrombectomy head is completely covered by the thrombus cutter in the axial direction.

4. The thrombus retrieval catheter of claim 2, wherein the axial length of the side wing is not less than the axial length of the thrombus cutter.

5. The thrombus retrieval catheter of claim 2, wherein the number of the side wings is equal to the number of thrombectomy holes.

6. The thrombus retrieval catheter of claim 2, wherein the side wing provided on the blocking tube is a long and narrow sheet.

7. The thrombus retrieval catheter of claim 2, wherein the side wing is a spiral side wing (1610, 1620), and a cutting edge (1611, 1621) is provided on the spiral side wing.

8. The thrombus retrieval catheter of claim 1, wherein a vacuum suction device is connected with the side hole, and a motor is connected with the proximal end of the transmission cable.

9. The thrombus retrieval catheter of claim 1, wherein a hydrophilic coating is covered over the outer surface of the blocking tube.

10. The thrombus retrieval catheter of claim 1, wherein the angle of the thrombectomy hole is 60~120° for the retrieval catheter used in artery, and the angle of the thrombectomy hole is 30∼60° for the retrieval catheter used in vein.

11. The thrombus retrieval catheter of claim 1, wherein a guide wire lumen (1820, 1920) through which a guide wire can pass is correspondingly provided on the thrombectomy head and outer sheath.

## Patentansprüche

1. Thrombusbergungskatheter (1000), umfassend einen Thrombektomiekopf (1100), einen Thrombusschneider (1200), ein Übertragungskabel (1500), eine Außenumhüllung (1300) und ein Endverbindungsteil (1400);
wobei der Thrombektomiekopf eine hohle röhrenförmige Struktur und mindestens ein Thrombektomieloch (1110, 1111, 1112) aufweist;
wobei der Thrombusschneider eine weitere hohle röhrenförmige Struktur aufweist, die mindestens eine Klinge (1210) aufweist;
wobei der Thrombusschneider innerhalb des Thrombektomiekopfes installiert ist; der Thrombusschneider mit einem distalen Ende des Übertragungskabels fest verbunden ist; ein proximales Ende des Thrombektomiekopfes mit einem distalen Ende der Außenumhüllung fest verbunden ist; ein proximales Ende der Außenumhüllung mit dem Endverbindungsteil fest verbunden ist; das Endverbindungsteil mit einem Seitenloch (1410) bereitgestellt ist; **dadurch gekennzeichnet, dass** der Katheter ferner ein Blockierungsrohr (1700, 1710, 1720) umfasst, das Blockierungsrohr als eine Hülse an der Außenseite des Übertragungskabels bereitgestellt ist; ein proximales Ende des Blockierungsrohrs mit dem Endverbindungsteil fest verbunden ist; der Thrombusschneider als eine Hülse auf der Außenseite des Blockierungsrohrs bereitgestellt ist; und wobei das Seitenloch mit einem Lumen in Kommunikation steht, das zwischen dem Blockierungsrohr und der Außenumhüllung ausgebildet ist.

2. Thrombusbergungskatheter nach Anspruch 1, wobei mindestens ein Seitenflügel (1600) an dem distalen Ende des Blockierungsrohrs fest bereitgestellt ist, wobei der Thrombusschneider als eine Hülse an der Außenseite des Seitenflügels bereitgestellt ist.

3. Thrombusbergungskatheter nach Anspruch 1, wobei das Thrombektomieloch an dem Thrombektomiekopf in der axialen Richtung durch den Thrombusschneider vollständig bedeckt ist.

4. Thrombusbergungskatheter nach Anspruch 2, wobei die axiale Länge des Seitenflügels nicht kleiner als die axiale Länge des Thrombusschneiders ist.

5. Thrombusbergungskatheter nach Anspruch 2, wobei die Anzahl der Seitenflügel gleich der Anzahl der Thrombektomielöcher ist.

6. Thrombusbergungskatheter nach Anspruch 2, wobei an dem Seitenflügel, der an dem Blockierungsrohr bereitgestellt ist, eine lange und schmale Bahn ist.

7. Thrombusbergungskatheter nach Anspruch 2, wobei der Seitenflügel ein spiralförmiger Seitenflügel (1610, 1620) ist und eine Schneidkante (1611, 1621) an dem spiralförmigen Seitenflügel bereitgestellt ist.

8. Thrombusbergungskatheter nach Anspruch 1, wobei eine Vakuumsaugvorrichtung mit dem Seitenloch verbunden ist und ein Motor mit dem proximalen Ende des Übertragungskabels verbunden ist.

9. Thrombusbergungskatheter nach Anspruch 1, wobei eine hydrophile Beschichtung über die äußere Oberfläche des Blockierungsrohrs überdeckt ist.

10. Thrombusbergungskatheter nach Anspruch 1, wobei der Winkel des Thrombektomielochs 60 ~ 120° für den in einer Arterie verwendeten Bergungskatheter beträgt und der Winkel des Thrombektomielochs 30 ~ 60° für den in einer Vene verwendeten Bergungskatheter beträgt.

11. Thrombusbergungskatheter nach Anspruch 1, wobei ein Führungsdrahtlumen (1820, 1920), durch das ein Führungsdraht passieren kann, auf dem Thrombektomiekopf und der Außenumhüllung entsprechend bereitgestellt ist.

## Revendications

1. Cathéter d'extraction de thrombus (1000), comprenant une tête de thrombectomie (1100), un dispositif de coupe de thrombus (1200), un câble de transmission (1500), une gaine externe (1300) et une partie de liaison d'extrémité (1400) ;
la tête de thrombectomie a une structure tubulaire creuse et au moins un trou de thrombectomie (1110, 1111, 1112) ;
le dispositif de coupe de thrombus a une structure tubulaire creuse supplémentaire qui a au moins une lame (1210) ;
le dispositif de coupe de thrombus est installé à l'intérieur de la tête de thrombectomie ; le dispositif de coupe de thrombus est relié de manière fixe à une extrémité distale du câble de transmission ; une extrémité proximale de la tête de thrombectomie est reliée de manière fixe à une extrémité distale de la gaine externe ; une extrémité proximale de la gaine externe est reliée de manière fixe à la partie de liaison d'extrémité ; la partie de liaison d'extrémité est fournie d'un trou latéral (1410) ; **caractérisé en ce que**, le cathéter comprend en outre un tube de blocage (1700, 1710, 1720), le tube de blocage étant fourni sous la forme d'un manchon à l'extérieur du câble de transmission ; une extrémité proximale du tube de blocage est reliée de manière fixe à la partie de liaison d'extrémité ; le dispositif de coupe de thrombus est fourni sous la forme d'un manchon à l'extérieur du tube de blocage ; et le trou latéral étant en communication avec une lumière formée entre le tube de blocage et la gaine externe.

2. Cathéter d'extraction de thrombus selon la revendication 1, dans lequel au moins une aile latérale (1600) est fournie de manière fixe sur l'extrémité distale du tube de blocage, le dispositif de coupe de thrombus étant fourni sous la forme d'un manchon sur l'extérieur de l'aile latérale.

3. Cathéter d'extraction de thrombus selon la revendication 1, dans lequel le trou de thrombectomie sur la tête de thrombectomie est complètement recouvert par le dispositif de coupe de thrombus dans la direction axiale.

4. Cathéter d'extraction de thrombus selon la revendication 2, dans lequel la longueur axiale de l'aile latérale n'est pas inférieure à la longueur axiale du dispositif de coupe de thrombus.

5. Cathéter d'extraction de thrombus selon la revendication 2, dans lequel le nombre des ailes latérales est égal au nombre de trous de thrombectomie.

6. Cathéter d'extraction de thrombus selon la revendication 2, dans lequel l'aile latérale fournie sur le tube de blocage est une feuille longue et étroite.

7. Cathéter d'extraction de thrombus selon la revendication 2, dans lequel l'aile latérale est une aile latérale en spirale (1610, 1620) et un bord coupant (1611, 1621) est fourni sur l'aile latérale en spirale.

8. Cathéter d'extraction de thrombus selon la revendication 1, dans lequel un dispositif d'aspiration à vide est relié au trou latéral et un moteur est relié à l'extrémité proximale du câble de transmission.

9. Cathéter d'extraction de thrombus selon la revendication 1, dans lequel un revêtement hydrophile est recouvert sur la surface extérieure du tube de blocage.

10. Cathéter d'extraction de thrombus selon la revendication 1, dans lequel l'angle du trou de thrombectomie est de 60 à 120 ° pour le cathéter d'extraction utilisé dans l'artère et l'angle du trou de thrombectomie est de 30 à 60 ° pour le cathéter d'extraction utilisé dans la veine.

11. Cathéter d'extraction de thrombus selon la revendication 1, dans lequel une lumière de fil de guidage (1820, 1920) à travers laquelle un fil de guidage peut passer est fournie en conséquence sur la tête de thrombectomie et la gaine externe.
